# EUROPEAN PATENT APPLICATION

(11) **EP 3 000 390 A1**
(43) Date of publication of application: **30.03.2016**
(21) Application number: 15174537.9
(22) Date of filing: 26.03.2008
(51) Int. Cl.: A61B 5/042, A61N 1/05

(54) **HIGH RESOLUTION ELECTROPHYSIOLOGY CATHETER**

(30) Priority: 26.03.2007 US 908166 P
(62) Divisional of application: 08744409.7
(71) Applicant: Boston Scientific Limited, Hamilton HM11 (BM)
(72) Inventor: Koblish, Josef V., Sunnyvale, CA 94087 (US)
(74) Representative: Pfenning, Meinig & Partner mbB

(57) **Abstract**

An electrophysiology medical probes, which may be incorporated into a system and used to perform an electrophysiology procedure, is provided. The medical probe comprises an elongated member (e.g., a flexible elongated member), and a metallic electrode mounted to the distal end of the elongated member. In one embodiment, the metallic electrode is cylindrically shaped and comprises a rigid body. The medical probe further comprises a plurality of microelectrodes (e.g., at least four microelectrodes) embedded within, and electrically insulated from, the metallic electrode, and at least one wire connected to the metallic electrode and the microelectrodes.

## Description

### FIELD OF THE INVENTION

The invention relates to systems and methods for providing therapy to a patient, and more particularly to systems and methods for mapping and ablating tissue within the heart of the patient

### BACKGROUND OF THE INVENTION

Physicians make use of catheters today in medical procedures to gain access into interior regions of the body to ablate targeted tissue regions. It is important for the physician to be able to precisely locate the catheter and control its emission of energy within the body during these tissue ablation procedures. For example, in electrophysiological therapy, ablation is used to treat cardiac rhythm disturbances in order to restore the normal function of the heart.

Normal sinus rhythm of the heart begins with the sinoatrial node (or "SA node") generating a depolarization wave front that propagates uniformly across the myocardial tissue of the right and left atria to the atrioventricular node (or "AV node"). This propagation causes the atria to contract in an organized manner to transport blood from the atria to the ventricles. The AV node regulates the propagation delay to the atrioventricular bundle (or "HIS" bundle), after which the depolarization wave front propagates uniformly across the myocardial tissue of the right and left ventricles, causing the ventricles to contract in an organized manner to transport blood out of the heart. This conduction system results in the described, organized sequence of myocardial contraction leading to a normal heartbeat.

Sometimes, aberrant conductive pathways develop in heart tissue, which disrupt the normal path of depolarization events. For example, anatomical obstacles in the atria or ventricles can disrupt the normal propagation of electrical impulses. These anatomical obstacles (called "conduction blocks") can cause the depolarization wave front to degenerate into several circular wavelets that circulate about the obstacles. These wavelets, called "reentry circuits," disrupt the normal activation of the atria or ventricles. As a further example, localized regions of ischemic myocardial tissue may propagate depolarization events slower than normal myocardial tissue. The ischemic region, also called a "slow conduction zone," creates errant, circular propagation patterns, called "circus motion." The circus motion also disrupts the normal depolarization patterns, thereby disrupting the normal contraction of heart tissue. The aberrant conductive pathways create abnormal, irregular, and sometimes life-threatening heart rhythms, called arrhythmias. An arrhythmia can take place in the atria, for example, as in atrial tachycardia (AT), atrial fibrillation (AFIB), or atrial flutter (AF). The arrhythmia can also take place in the ventricle, for example, as in ventricular tachycardia (VT).

In treating these arrhythmias, it is essential that the location of the sources of the aberrant pathways (called substrates) be located. Once located, the tissue in the substrates can be destroyed, or ablated, by heat, chemicals, or other means of creating a lesion in the tissue, or otherwise can be electrically isolated from the normal heart circuit Electrophysiology therapy involves locating the aberrant pathways via a mapping procedure, and forming lesions by soft tissue coagulation on the endocardium (the lesions being 1 to 15 cm in length and of varying shape) using an ablation catheter to effectively eliminate the aberrant pathways. In certain advanced electrophysiology procedures, as part of the treatment for certain categories of atrial fibrillation, it may be desirable to create a curvilinear lesion around or within the ostia of the pulmonary veins (PVs), and a linear lesion connecting one or more of the PVs to the mitral valve annulus. Preferably, such curvilinear lesion is formed as far out from the PVs as possible to ensure that the conduction blocks associated with the PVs are indeed electrically isolated from the active heart tissue.

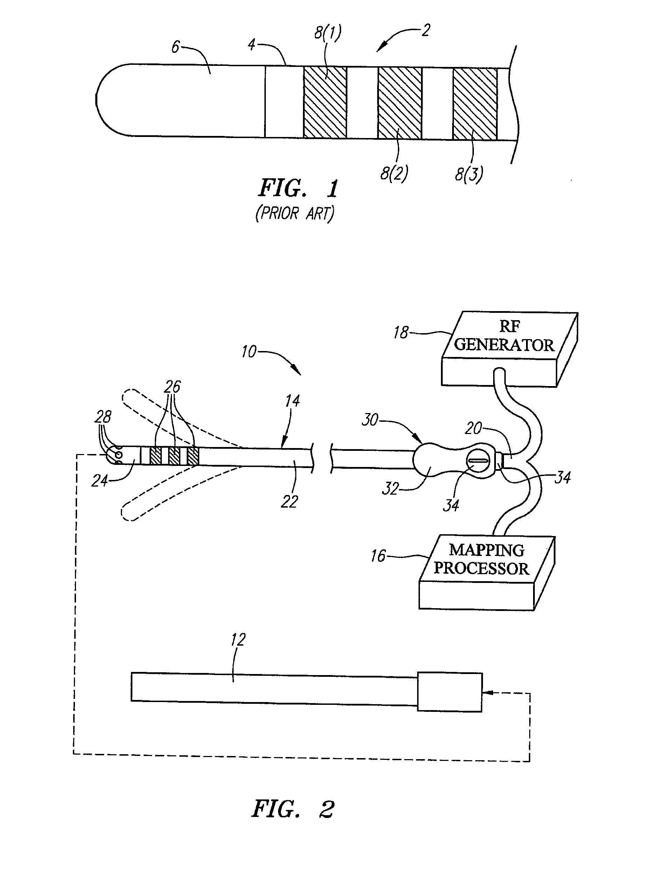

Referring to Fig. 1, a prior art electrophysiological catheter 2 includes a flexible catheter body 4, a tip electrode 6 mounted to the distal end of the catheter body 4, and a plurality of ring electrodes 8 (distal ring electrode 8(1), medial ring electrode 8(2), and proximal ring electrode 8(3)) mounted to the distal end of the catheter body 4 proximal to the tip electrode 6. In this embodiment, the tip electrode 6 serves as both a tissue ablation electrode and a tissue mapping electrode, and the ring electrodes 8 serve as dedicated mapping electrodes. In a typical mapping procedure, the tip electrode 6, and if possible the ring electrodes 8, are placed into contact with the endocardial tissue of the heart chamber stricken with the arrhythmia to obtain multiple electrocardiograms (ECGs) or monophasic action potentials (MAPs) by measuring electrical signals at the electrodes 6, 8. For example, three bipolar ECG recordings may be obtained by measuring the voltage potentials between various pairs of the electrodes (e.g., between the tip electrode 6 and the distal ring electrode 8(1), between the distal ring electrode 8(1) and the medial ring electrode 8(2), or between the medial ring electrode 8(2) and the proximal ring electrode 8(3)).

Based on the ECG or MAP recordings, the physician can determine the relative location of the catheter in the heart and/or the location of any aberrant pathways. In one technique, the morphologies of the ECG or MAP recordings, themselves, can be analyzed by a physician to determine the relative location of the catheter in the heart. In another technique, the electrode recordings are processed to generate isochronal electrophysiology maps, which may be combined with three-dimensional anatomical maps, such as those generated in three-dimensional medical systems (e.g., the Realtime Position Management (RPM) tracking system, developed commercially by Boston Scientific Corporation and described in U.S. Pat. Nos. 6,216,027 and 6,950,689, and the CARTO EP Medical system, developed commercially by Biosense Webster and described in U.S. Pat No. 5,391,199).

Primarily due to the relatively large size of tip electrodes, current catheter designs, such as the type illustrated in Fig. 1, may detect far field electrical activity (i.e., the ambient electrical activity away from the recording electrode(s)), which can negatively affect the detection of local electrical activity. That is, due to the relatively large size of the tip electrode and the distance from the next ring electrode, the resulting electrical recordings are signal averaged and blurred, and thus not well-defined. This far-field phenomenon becomes more exaggerated, thereby decreasing the mapping resolution, as the length of distal tip electrode increases.

Thus, the electrical activity measured by such catheters does not always provide a physician with enough resolution to accurately identify an ablation site and or provide the physician with an accurate portrayal of the real position of the tip electrode, thereby causing the physician to perform multiple ablations in several areas, or worse yet, to perform ablations in locations other than those that the physician intends.

In addition, many significant aspects of highly localized electrical activity may be lost in the far-field measurement. For example, the high frequency potentials that are encountered around pulmonary veins or fractioned ECGs associated with atrial fibrillation triggers may be lost. Also, it may be difficult to determine the nature of the tissue with which the tip electrode is in contact, or whether the tip electrode is in contact with tissue at all, since the far-field measurements recorded by the tip electrode may indicate electrical activity within the myocardial tissue even though the tip electrode is not actually in contact with the endocardial tissue.

For example, it may be very important to ascertain whether the tip electrode is in contact with endocardial tissue or venous tissue during an ablation procedure. This becomes especially significant when ablating in and around the ostia of the pulmonary veins, since ablation within the pulmonary veins, themselves, instead of the myocardial tissue, may cause stenosis of the pulmonary veins. However, the far field measurements taken by the tip electrode may indicate that the tip electrode is in contact with endocardial tissue, when in fact, the tip electrode is in contact with venous tissue. As another example, it may be desirable to ascertain lesion formation by measuring the electrical activity of the tissue in contact with the tip electrode (i.e., the lack of electrical activity indicates ablated tissue, whereas the presence of electrical activity indicates live tissue). However, due to the far-field measurements, electrical activity may be measured from nearby live tissue, even though the tip electrode is actually in contact with ablated tissue.

Accordingly, there remains a need for an electrophysiology catheter that is capable of measuring electrical activity of tissue at a higher resolution.

### SUMMARY OF THE INVENTION

In accordance with first embodiment of the invention, a medical probe comprises an elongated member (e.g., a flexible elongated member), and a metallic electrode mounted to the distal end of the elongated member. In one embodiment, the metallic electrode is cylindrically shaped and comprises a rigid body. The medical probe further comprises a plurality of microelectrodes (e.g., at least four microelectrodes) embedded within, and electrically insulated from, the metallic electrode, and at least one wire connected to the metallic electrode and the microelectrodes. Each microelectrode may have a suitably small size, e.g., less than 2mm. The exterior surfaces of the microelectrodes may conform to an exterior surface of the metallic electrode to form an electrode assembly with a substantially continuous exterior surface.

In one embodiment, the metallic electrode has a cylindrical wall, a bore surrounded by the cylindrical wall, and a plurality of holes extending through the cylindrical wall in communication with the bore. In this case, the microelectrodes are respectively disposed within the holes. The distal end of the elongated member may be disposed within the bore of the metallic electrode, and the medical probe may further comprise an electrically insulative potting material disposed within the bore. In this embodiment, the medical probe may further comprise a plurality of electrically insulative bands respectively disposed within the holes, in which case, the microelectrodes are respectively disposed within the electrically insulative bands.

In accordance with a a second embodiment of the invention, a medical probe comprises an elongated member (e.g., a flexible elongated member), and a cap electrode mounted to the distal tip of the elongated member. In one embodiment, the cap electrode has a length equal to or greater than 4mm and is composed of a metallic material. The medical probe further comprises a plurality of microelectrodes (e.g., at least four microelectrodes) disposed on, and electrically insulated from, the cap electrode, and at least one wire connected to the cap electrode and the microelectrodes. The cap electrode and microelectrodes may be integrated together in the same manner as the metallic electrode and microelectrodes described above. In one embodiment, the medical probe further comprises at least one ring electrode, mounted around the elongated member proximal to the cap electrode, in which case, the wire(s) is connected to the ring electrode(s).

In accordance with a third embodiment of the invention, a medical probe comprises an elongated member (e.g., a flexible elongated member), and a rigid electrode mounted to the distal end of the elongated member. In one embodiment, the metallic electrode is cylindrically shaped and is composed of a metallic material. The medical probe further comprises a plurality of microelectrodes (e.g., at least four microelectrodes) disposed on, and electrically insulated from, the cap electrode, and at least one wire connected to the cap electrode and the microelectrodes. The rigid electrode and microelectrodes may be integrated together in the same manner as the metallic electrode and microelectrodes described above.

In accordance with a fourth embodiment of the invention, a medical system comprises any of the medical probes described above, a radio frequency (RF) ablation source coupled to the wire(s), and a mapping processor coupled to the wire(s).

In accordance with a fifth embodiment of the invention, a medical method comprises using any of the medical probes described above into a patient for purpose of better understanding the invention. The method further comprises placing the metallic electrode, cap electrode, or rigid electrode into contact with tissue (e.g., cardiac tissue) within the patient, sensing the tissue via at least one of the microelectrodes, and conveying ablation energy from the metallic electrode, cap electrode, or rigid electrode to ablate the tissue. In one method, the medical probe is intravenously introduced into the patient, in which case, the cardiac tissue may be endocardial tissue.

In accordance with a sixth embodiment of the invention a method of manufacturing a medical probe comprises providing a cylindrically-shaped electrode having a wall and a bore surrounded by the wall. The method further comprises forming a plurality of holes through the wall into the bore (e.g., by drilling the holes), mounting a plurality of microelectrodes (e.g., at least four microelectrodes) respectively into the holes, mounting the distal end of an elongated member (e.g., a flexible elongated member) into the bore, connecting at least one wire to the electrode and microelectrodes and disposing the wire(s) through the elongated member.

In one method, the electrode has a hemi-spherical distal tip, in which case, the distal tip of the elongated member is mounted into the bore. One method further comprises mounting a plurality of electrically insulative bands respectively into the holes, in which case, the microelectrodes are respectively mounted within the electrically insulative bands. In one method, each of the microelectrodes has a diameter equal to or less than 4mm. Another method further comprises introducing an electrically insulative potting material within the bore prior to mounting the distal end of the elongated member within the bore. Still another method further comprises grinding an exterior surface of the electrode and the exterior surfaces of the microelectrodes to form an electrode assembly with a substantially continuous exterior surface.

The use of microelectrodes in the manner described above eliminates detection of the far field electrical activity, thereby increasing the resolution and fidelity of the mapping performed by the medical probe, allowing a user to more precisely measure complex localized electrical activity, and more accurately detecting tissue contact and tissue characterization, including lesion formation assessment.

In the following several aspects of the present invention are described:
1. A medical probe, comprising:
   an elongated member having a distal end;
   a metallic electrode mounted to the distal end of the elongated member;
   a plurality of microelectrodes embedded within, and electrically insulated from the metallic electrode; and
   at least one wire extending through the elongated member and connected tc the metallic electrode and the microelectrodes.
2. The medical probe of aspect 1, wherein the elongated member is flexible.
3. The medical probe of aspect 1, wherein the metallic electrode comprises a rigid body.
4. The medical probe of aspect 1, metallic electrode is cylindrically-shaped.
5. The medical probe of aspect 1, wherein the plurality of microelectrodes comprises at least four microelectrodes
6. The medical probe of aspect 1, wherein each of the microelectrodes has a diameter equal to or less than 4mm.
7. The medical probe of aspect 1, wherein exterior surfaces of the microelectrodes conform to an exterior surface of the metallic electrode to form an electrode assembly with a substantially continuous exterior surface.
8. The medical probe of aspect 1, wherein the metallic electrode has a cylindrical wall, a bore surrounded by the cylindrical wall, and a plurality of holes extending through the cylindrical wall in communication with the bore, and wherein the microelectrodes are respectively disposed within the holes.
9. The medical probe of aspect 8, wherein the distal end of the elongated member is disposed within the bore of the metallic electrode.
10. The medical probe of aspect 8, further comprising a plurality of electrically insulative bands respectively disposed within the holes, wherein the microelectrodes are respectively disposed within the electrically insulative bands.
11. The medical probe of aspect 8, further comprising an electrically insulative potting material disposed within the bore.
12. A medical system, comprising:
   the medical probe of aspect 1;
   a radio frequency (RF) ablation source coupled to the one wire; and
   a mapping processor coupled to the at least other wire.
13. A medical method comprising:
   introducing the medical probe of aspect 1 into a patient;
   placing the metallic electrode into contact with tissue within the patient;
   sensing the tissue via at least one of the microelectrodes; and
   conveying ablation energy from the metallic electrode to ablate the tissue.
14. The method of aspect 13, wherein the tissue is cardiac tissue.
15. The method of aspect 14, wherein the medical probe is intravenously introduced into the patient, and the cardiac tissue is endocardial tissue.
16. A medical probe, comprising:
   an elongated member having a distal tip;
   a cap electrode mounted to the distal tip of the elongated member;
   a plurality of microelectrodes disposed on, and electrically insulated from, the cap electrode; and
   at least one wire extending through the elongated member and connected to the cap electrode and the microelectrodes.
17. The medical probe of aspect 16, wherein the elongated member is flexible.
18. The medical probe of aspect 16, wherein the cap electrode comprises a rigid body.
19. The medical probe of aspect 16, wherein the cap electrode is composed of a metallic material.
20. The medical probe of aspect 16, wherein the cap electrode has a length equal to or greater than 4mm.
21. The medical probe of aspect 16, wherein the plurality of microelectrodes comprises at least four microelectrodes.
22. The medical probe of aspect 16, wherein each of the microelectrodes has a diameter equal to or less than 4mm.
23. The medical probe of aspect 16, wherein exterior surfaces of the microelectrodes conform to an exterior surface of the cap electrode to form an electrode assembly with a substantially continuous exterior surface.
24. The medical probe of aspect 16, wherein the microelectrodes are embedded within the cap electrode.
25. The medical probe of aspect 16, wherein the cap electrode has a cylindrical wall, a bore surrounded by the cylindrical wall, and a plurality of holes extending through the cylindrical wall in communication with the bore, and wherein the microelectrodes are respectively disposed within the holes.
26. The medical probe of aspect 25, wherein the distal tip of the elongated member is disposed within the bore of the cap electrode.
27. The medical probe of aspect 25, further comprising a plurality of electrically insulative bands respectively disposed within the holes, wherein the microelectrodes are respectively disposed within the electrically insulative bands.
28. The medical probe of aspect 25, further comprising an electrically insulative potting material disposed within the bore.
29. The medical probe of aspect 16, further comprising at least one ring electrode mounted around the elongated member proximal to the cap electrode, wherein the at least one wire is connected to the at least one ring electrode.
30. A medical system, comprising:
   the medical probe of aspect 16;
   a radio frequency (RF) ablation source coupled to the one wire; and
   a mapping processor coupled to the at least other wire.
31. A medical method comprising:
   introducing the medical probe of aspect 16 into a patient;
   placing the cap electrode into contact with tissue within the patient;
   sensing the tissue via at least one of the microelectrodes; and
   conveying ablation energy from the metallic electrode to ablate the tissue.
32. The method of aspect 31, wherein the tissue is cardiac tissue.
33. The method of aspect 32, wherein the medical probe is intravenously introduced into the patient, and the cardiac tissue is endocardial tissue.
34. A medical probe, comprising:
   an elongated member having a distal end;
   a rigid electrode mounted to the distal end of the elongated member;
   a plurality of microelectrodes disposed on, and electrically insulated from, the rigid electrode; and
   at least one wire extending through the elongated member and connected to the rigid electrode and the microelectrodes
35. The medical probe of aspect 34, wherein the elongated member is flexible.
36. The medical probe of aspect 34, wherein the rigid electrode is composed of a metallic material.
37. The medical probe of aspect 34, wherein the rigid electrode is cylindrically-shaped.
38. The medical probe of aspect 34, wherein the plurality of microelectrodes comprises at least four microelectrodes.
39. The medical probe of aspect 34, wherein each of the microelectrodes has a diameter equal to or less than 4mm.
40. The medical probe of aspect 34, wherein exterior surfaces of the microelectrodes conform to an exterior surface of the rigid electrode to form an electrode assembly with a substantially continuous exterior surface.
41. The medical probe of aspect 34, wherein the microelectrodes are embedded within the rigid electrode.
42. The medical probe of aspect 34, wherein the rigid electrode has a cylindrical wall, a bore surrounded by the cylindrical wall, and a plurality of holes extending through the cylindrical wall in communication with the bore, and wherein the microelectrodes are respectively disposed within the holes.
43. The medical probe of aspect 42, wherein the distal end of the elongate member is disposed within the bore of the rigid electrode.
44. The medical probe of aspect 42, further comprising a plurality of electrically insulative bands respectively disposed within the holes, wherein the microelectrodes are respectively disposed within the electrically insulative bands.
45. The medical probe of aspect 42, further comprising an electrically insulative potting material disposed within the bore.
46. A medical system, comprising:
   the medical probe of aspect 34;
   a radio frequency (RF) ablation source coupled to the one wire; and
   a mapping processor coupled to the at least other wire.
47. A medical method comprising:
   introducing the medical probe of aspect 34 into a patient;
   placing the rigid electrode into contact with tissue within the patient;
   sensing the tissue via at least one of the microelectrodes; and
   conveying ablation energy from the rigid electrode to ablate the tissue.
48. The method of aspect 47, wherein the tissue is cardiac tissue.
49. The method of aspect 48, wherein the medial probe is intravenously introduced into the patient, and the cardiac tissue is endocardial tissue.
50. A method of manufacturing a medical probe, comprising:
   providing a cylindrically-shaped electrode having a wall and a bore surrounded by the wall;
   forming a plurality of holes through the wall into the bore;
   mounting a plurality of microelectrodes respectively into the holes;
   mounting a distal end of an elongated member into the bore;
   connecting at least one wire to the electrode and the microelectrodes; and
   disposing the at least one wire through the elongated member.
51. The method of aspect 50, wherein the electrode has a hemi-spherical distal tip, and wherein a distal tip of the elongated member is mounted into the bore.
52. The method of aspect 50, wherein the holes are drilled through the wall into the bore.
53. The method of aspect 50, wherein the elongated member is flexible.
54. The method of aspect 50, wherein the plurality of microelectrodes comprises at least four microelectrodes.
55. The method of aspect 50, further comprising mounting a plurality of electrically insulative bands respectively into the holes, wherein the microelectrodes are respectively mounted within the electrically insulative bands.
56. The method of aspect 50, further comprising introducing an electrically insulative potting material within the bore prior to mounting the distal end of the elongated member within the bore.
57. The method of aspect 50, wherein each of the microelectrodes has a diameter equal to or less than 4mm.
58. The method of aspect 50, further comprising grinding an exterior surface of the electrode and the exterior surfaces of the microelectrodes to form an electrode assembly with a substantially continuous exterior surface.

### BRIEF DESCRIPTION OF THE DRAWINGS

The drawings illustrate the design and utility of embodiments of the invention, in which similar elements are referred to by common reference numerals, and in which:
Fig. 1 is a partially cutaway plan view of a prior art electrophysiology catheter;
Fig. 2 is a plan view of one embodiment of an electrophysiology system constructed in accordance with the invention;
Fig. 3 is a partially cutaway plan view of an electrophysiology catheter used in the system of Fig. 2, particularly showing a first arrangement of microelectrodes;
Fig. 4 is a cross-sectional view of the electrophysiology catheter of Fig. 3, taken along the line 4-4;
Fig. 5 is a cross-sectional view of one microelectrode incorporated into the electrophysiology catheter of Fig. 3;
Fig. 6 is a partially cutaway plan view of the electrophysiology catheter of Fig. 3, particularly showing a second arrangement of microelectrodes;
Fig. 7 is a partially cutaway plan view of the electrophysiology catheter of Fig. 3, particularly showing a third arrangement of microelectrodes;
Fig. 8 is a partially cutaway plan view of the electrophysiology catheter of Fig. 3, particularly showing a fourth arrangement of microelectrodes;
Fig. 9 is a partially cutaway plan view of the electrophysiology catheter of Fig. 3, particularly showing a fifth arrangement of microelectrodes;
Fig. 10 is a distal view of the electrophysiology catheter of Fig. 3;
Fig. 11 is a cross-sectional view of another microelectrode incorporated into the electrophysiology catheters of Figs. 5 and 6;
Figs. 12A-12C are plan views of a method of using the electrophysiology system of Fig. 2 to map and create lesions within the left atrium of a heart
Fig. 13 is a diagram illustrating electrocardiograms generated by the electrophysiology system of Fig. 2, particularly when the distal end of the electrophysiology catheter is slowly placed into firm contact witch endocardial tissue;
Fig. 14 is a diagram illustrating electrocardiograms generated by the electrophysiology system of Fig. 2, particularly when the distal end of the electrophysiology catheter is removed from a superior vena cava into contact with endocardial tissue;
Fig. 15 is a diagram illustrating electrocardiograms generated by the electrophysiology system of Fig. 2, particularly when RF ablation energy is delivered from the electrophysiology catheter into endocardial tissue; and
Fig. 16 is a diagram illustrating electrocardiograms generated by the electrophysiology system of Fig. 2, particularly when the distal end of the electrophysiology catheter is placed into contact with the left ventricle of a heart adjacent the atrioventricular node.

### DETAILED DESCRIPTION OF THE ILLUSTRATED EMBODIMENTS

Referring to Fig. 2, an exemplary electrophysiology system 10 constructed in accordance with the invention is shown. The system 10 may be used within body lumens, chambers or cavities of a patient for therapeutic and diagnostic purposes in those instances where access to interior bodily regions Is obtained through, for example, the vascular system or alimentary canal and without complex invasive surgical procedures. For example, the system 10 has application in the diagnosis and treatment of arrhythmia conditions within the heart. The system 10 also has application in the treatment of aliments of the gastrointestinal tract, prostrate, brain, gall bladder, uterus, and other regions of the body. As an example, the system 10 will be described hereinafter for use in the heart for mapping and ablating arrhythmia substrates.

The system 10 generally comprises a conventional guide sheath 12, and an electrophysiology catheter 14 that can be guided through a lumen (not shown) in the guide sheath 12. As will be described in further detail below, the electrophysiology catheter 14 is configured to be introduced through the vasculature of the patient, and into one of the chambers of the heart, where it can be used to map and ablate myocardial tissue. The system 10 also comprises a mapping processor 16 and a source of ablation energy, and in particular, a radio frequency (RF) generator 18, coupled to the electrophysiology catheter 14 via a cable assembly 20. Although the mapping processor 16 and RF generator 18 are shown as discrete components, they can alternatively be incorporated into a single integrated device.

The mapping processor 16 is configured to detect, process, and record electrical signals within the heart via the electrophysiology catheter 14. Based on these electrical signals, a physician can identify the specific target tissue sites within the heart, and ensure that the arrhythmia causing substrates have been electrically isolated by the ablative treatment. Based on the detected electrical signals, the mapping processor 16 outputs electrocardiograms (ECGs) to a display (not shown), which can be analyzed by the user to determine the existence and/or location of arrhythmia substrates within the heart and/or determine the location of the electrophysiology catheter 14 within the heart. In an optional embodiment, the mapping processor 16 can generate and output an isochronal map of the detected electrical activity to the display for analysis by the user. Such mapping techniques are well known in the art, and thus for purposes of brevity, will not be described in further detail.

The RF generator 18 is configured to deliver ablation energy to the electrophysiology catheter 14 in a controlled manner in order to ablate the target tissue sites identified by the mapping processor 16. Ablation of tissue within the heart is well known in the art, and thus for purposes of brevity, the RF generator 18 will not be described in further detail. Further details regarding RF generators are provided in U.S. Patent No. 5,383,874.

The electrophysiology catheter 14 may be advanced though the guide sheath 12 to the target location. The sheath 12, which should be lubricious to reduce friction during movement of the electrophysiology catheter 14, may be advanced over a guidewire in conventional fashion. Alternatively, a steerable sheath may be provided. With respect to materials, the proximal portion of the sheath 12 is preferably a Pebax® material and stainless steel braid composite, and the distal portion is a more flexible material, such as unbraided Pebax®, for steering purposes. The sheath 12 should also be stiffer than the electrophysiology catheter 14. A sheath introducer (not shown), such as those used in combination with basket catheters, may be used when introducing the electrophysiology catheter 14 into the sheath 12. The guide sheath 12 preferably includes a radio-opaque compound, such as barium, so that the guide sheath 12 can be observed using fluoroscopic or ultrasound imaging, or the like. Alternatively, a radio-opaque marker (not shown) can be placed at the distal end of the guide sheath 12.

The electrophysiology catheter 14 comprises an integrated flexible catheter body 22, a plurality of distally mounted electrodes, and in particular, a tissue ablation electrode 24, a plurality of mapping ring electrodes 26, a plurality of mapping microelectrodes 28, and a proximally mounted handle assembly 30. In alternative embodiments, the flexible catheter 14 may be replaced with a rigid surgical probe if percutaneous introduction or introduction through a surgical opening within a patient is desired.

The handle assembly 30 comprises a handle 32 composed of a durable and rigid material, such as medical grade plastic, and ergonomically molded to allow a physician to more easily manipulate the electrophysiology catheter 14. The handle assembly 30 comprises an external connector 34, such as an external multiple pin connector, received in a port on the handle assembly 30 with which the cable assembly 20 mates, so that the mapping processor 16 and RF generator 18 can be functionally coupled to the electrophysiology catheter 14. The handle assembly 30 may also include a printed circuit (PC) board (not shown) coupled to the external connector 34 and contained within the handle 32. The handle assembly 30 further including a steering mechanism 34, which can be manipulated to bidirectionally deflect the distal end of the electrophysiology catheter 14 (shown in phantom) via steering wires (not shown). Further details regarding the use of steering mechanisms are described in U.S. Patent Nos. 5,254,088 and 6,579,278.

The catheter body 22 is preferably about 5 French to 9 French in diameter, and between 80cm to 150cm in length. The catheter body 22 preferably has a cross-sectional geometry that is circular. However, other cross-sectional shapes, such as elliptical, rectangular, triangular, and various customized shapes, may be used as well. The catheter body 22 is preferably preformed of an inert, resilient plastic material that retains its shape and does not soften significantly at body temperature; for example, Pebax®, polyethylene, or Hytrel® (polyester). Alternatively, the catheter body 22 may be made of a variety of materials, including, but not limited to, metals and polymers. The catheter body is preferably flexible so that it is capable of winding through a tortuous path that leads to a target site, i.e., an area within the heart. Alternatively, the catheter body 22 may be semi-rigid, i.e., by being made of a stiff material, or by being reinforced with a coating or coil, to limit the amount of flexing.

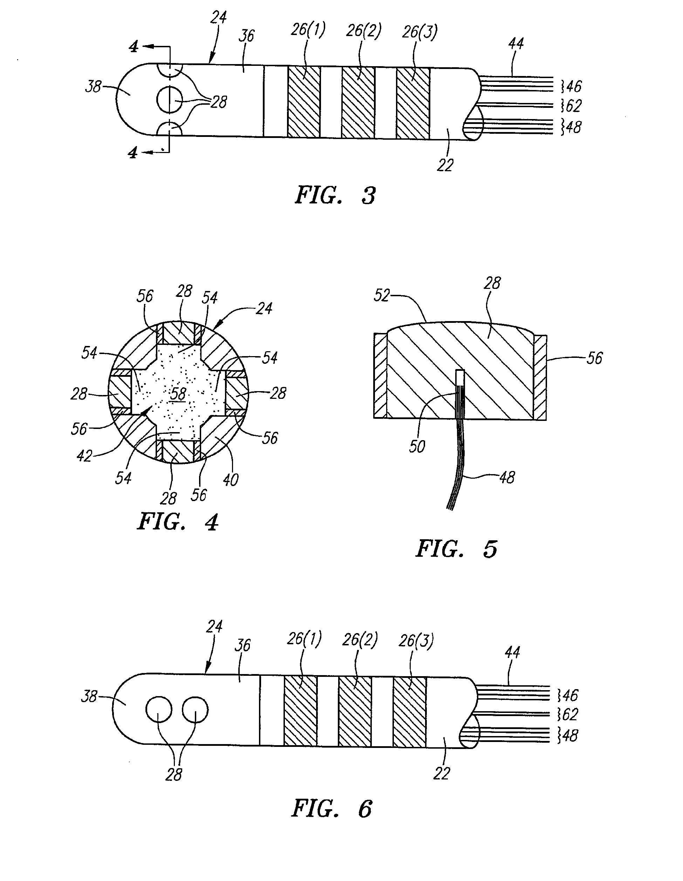

In the illustrated embodiment, the tissue ablation electrode 24 takes the form of a cap electrode mounted to the distal tip of the catheter body 22. In particular, and with further reference to Fig. 3, the ablation electrode 24 has a cylindrically-shaped proximal region 36 and a hemispherical distal region 38. As shown further in Fig. 4, the proximal region 36 of the ablation electrode 24 has a wall 40 and a bore 42 surrounded by the wall 40. The ablation electrode 24 may have any suitable length; for example, in the range between 4mm and 10mm. In the illustrated embodiment, the length of the ablation electrode 24 is 8mm. Preferably, the ablation electrode 24 is composed of a solid, electrically conductive material, such as platinum, gold, or stainless steel. The wall 40 of the ablation electrode 24 has a suitable thickness, such that the ablation electrode 24 forms a rigid body. For the purposes of this specification, an electrode is rigid if it does not deform when pressed into firm contact with solid tissue (e.g., cardiac tissue). The ablation electrode 24 is electrically coupled to the RF generator 18 (shown in Fig. 2), so that ablation energy can be conveyed from the RF generator 18 to the ablation electrode 24 to form lesions in myocardial tissue. To this end, an RF wire 44 (shown in Fig. 3) is electrically connected to the ablation electrode 24 using suitable means, such as soldering or welding. The wire 44 is passed in a conventional fashion through a lumen (not shown) extending through the associated catheter body 22, where it is electrically coupled either directly to the external connector 34 or indirectly to the external connector 34 via the PC board located in the handle assembly 30, which, in turn, is electrically coupled to the RF generator 18 via the cable assembly 20.

The mapping ring electrodes 26 include a distal mapping ring electrode 26(1), a medial mapping ring electrode 26(2), and a proximal mapping ring electrode 26(3). The mapping ring electrodes 26, as well as the tissue ablation electrode 24, are capable of being configured as bipolar mapping electrodes. In particular, the ablation electrode 24 and distal mapping ring electrode 26(1) can be combined as a first bipolar mapping electrode pair, the distal mapping ring electrode 26(1) and the medial mapping ring electrode 26(2) may be combined as a second bipolar mapping electrode pair, and the medial mapping ring electrode 26(2) and the proximal mapping ring electrode 26(3) may be combined as a third bipolar mapping electrode pair.

In the illustrated embodiment, the mapping ring electrodes 26 are composed of a solid, electrically conducting material, like platinum, gold, or stainless steel, attached about the catheter body 22. Alternatively, the mapping ring electrodes 26 can be formed by coating the exterior surface of the catheter body 22 with an electrically conducting material, like platinum or gold. The coating can be applied using sputtering, ion beam deposition, or equivalent techniques. The mapping ring electrodes 26 can have suitable lengths, such as between 0.5mm and 5mm. The mapping ring electrodes 26 are electrically coupled to the mapping processor 16 (shown in Fig. 2), so that electrical events in myocardial tissue can be sensed for the creation of electrograms or monophasic action potentials (MAPs), or alternatively, isochronal electrical activity maps. To this end, signal wires 46 (shown in Fig. 3) are respectively connected to the mapping ring electrodes 26 using suitable means, such as soldering or welding. The signal wires 46 are passed in a conventional fashion through a lumen (not shown) extending through the associated catheter body 22, where they are electrically coupled either directly to the external connector 34 or indirectly to the external connector 34 via the PC board located in the handle assembly 30, which, in turn, is electrically coupled to the mapping processor 16 via the cable assembly 20.

Like the mapping ring electrodes 26, the mapping microelectrodes 28 are electrically coupled to the mapping processor 16 (shown in Fig. 2), so that electrical events in myocardial tissue can be sensed for the creation of electrograms or MAPs, or alternatively, isochronal electrical activity maps. To this end, signal wires 48 (shown in Fig. 3) are respectively connected to the mapping microelectrodes 28 using suitable means, such as soldering or welding. The signal wires 48 are passed in a conventional fashion through a lumen (not shown) extending through the associated catheter body 22, where they are electrically coupled either directly to the external connector 34 or indirectly to the external connector 34 via the PC board located in the handle assembly 30, which, in turn, is electrically coupled to the mapping processor 16 via the cable assembly 20.

Significantly, the microelectrodes 28 are disposed on the tissue ablation electrode 24, and in particular, are embedded within the wall 40 of the tissue ablation electrode 24. This allows the localized intracardial electrical activity to be measured in real time at the point of energy delivery from the ablation electrode 24. In addition, due to their relatively small size and spacing, the microelectrodes 28 do not sense far field electrical potentials that would normally be associated with bipolar measurements taken between the tissue ablation electrode 24 and the mapping ring electrodes 26.

Instead, the microelectrodes 28 measure the highly localized electrical activity at the point of contact between the ablation electrode 24 and the endocardial tissue. Thus, the arrangement of the microelectrodes 28 substantially enhances the mapping resolution of the electrophysiology catheter 14. The high resolution inherent in the microelectrode arrangement will allow a user to more precisely measure complex localized electrical activity, resulting in a powerful tool for diagnosing ECG activity; for example, the high frequency potentials that are encountered around pulmonary veins or the fractioned ECGs associated with atrial fibrillation triggers.

Moreover, the microelectrode arrangement lends itself well to creating MAPs, which may play an important role in diagnosing AFIB triggers. In particular, a focal substrate may be mapped by the microelectrodes 28, and without moving the ablation electrode 24, the mapped focal substrate may be ablated. The microelectrode arrangement also allows for the generation of high density electrical activity maps, such as electrical activity isochronal maps, which may be combined with anatomical maps, to create electro-anatomical maps. In addition, due to the elimination or minimization of the detected far field electrical activity, detection of tissue contact and tissue characterization, including lesion formation assessment, is made more accurate.

The microelectrodes 28 may be disposed on the ablation electrode 24 in any one of a variety of different patterns. In the embodiment illustrated in Fig. 3, four microelectrodes 28 (only three shown) are circumferentially disposed about the cylindrical-shaped region 36 of the ablation electrode 24 at ninety degree intervals, so that they face radially outward in four different directions. In another embodiment illustrated in Fig. 6, four microelectrodes 28 are arranged into two longitudinally disposed pairs (only pair shown) circumferentially disposed about the cylindrical-shaped proximal region 36 of the ablation electrode 24 at a one hundred degree interval, so that the electrode pairs face radially outward in two opposite directions.

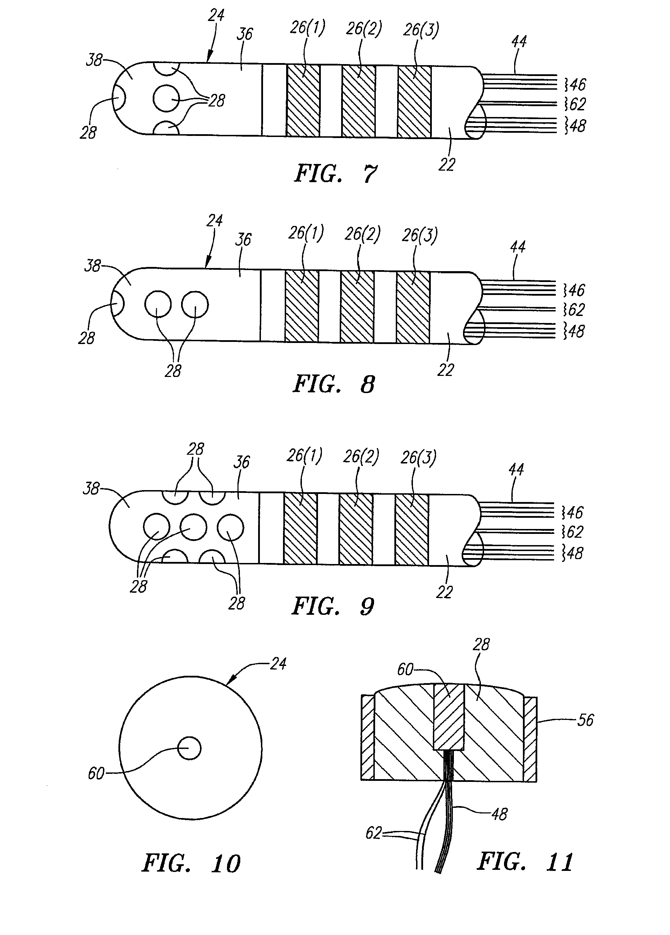

Other embodiments illustrated in Figs. 7 and 8, are respectively similar to the embodiments illustrated in Figs. 5 and 6, with the exception that a fifth microelectrode 28 is disposed on the hemispherical distal region 38 of the ablation electrode 24, so that it faces distally outward. In yet another embodiment, as shown in Fig. 9, ten microelectrodes 28 are arranged into two longitudinally disposed trios (only one shown) and two longitudinally disposed pairs circumferentially disposed about the cylindrical-shaped proximal region 36 of the ablation electrode 24 at ninety degree intervals, so that the electrode trios and pairs face radially outward in four different directions. Notwithstanding the different microelectrode patterns, as a general rule, it is preferable that the microelectrodes 28 be located as distal on the ablation electrode 24 as possible. In this manner, the microelectrodes 28 will be placed into contact with tissue when the distal end of the electrophysiology catheter 14 is oriented perpendicularly to the tissue.

In the illustrated embodiments, each of the microelectrodes 28 has a circular profile for ease of manufacture, although in alternative embodiments, the microelectrodes 28 may have other profiles, such as elliptical, oval, or rectangular. The microelectrodes 28 have relatively small diameters and are spaced a relatively small distance from each other in order to maximize the mapping resolution of the microelectrodes 28, as will be described in further detail below. Ultimately, the size and spacing of the microelectrodes 28 will depend upon the size of the ablation electrode 24, as well as the number and particular pattern of the microelectrodes 28. Preferably, the diameter of each microelectrode 28 is equal to or less than half the length of the ablation electrode 24, and more preferably equal to or less than one-quarter the length of the ablation electrode 24. For example, if the length of the ablation electrode 24 is 8mm, the diameter of each microelectrode 28 may be equal to or less than 4mm, and preferably equal to or less than 2mm. The spacing of the microelectrodes 28 (as measured from center to center) may be equal to or less than twice the diameter, and preferably equal to or less than one and half times the diameter of each microelectrode 28.

Each microelectrode 28 is composed of an electrically conductive material, such as platinum, gold, or stainless steel, but preferably is composed of a silver/silver chloride to maximize the coupling between the microelectrode 28 and blood, thereby optimizing signal fidelity. As shown in Fig. 5, each microelectrode 28 is substantially solid, having a small bore 50 formed in one end of the microelectrode 28 along its axis, thereby providing a convenient means for connecting a signal wire 48 to the microelectrode 28 via suitable means, such as soldering or welding.

Each microelectrode 28 also has a tissue-contacting surface 52 opposite the bore 42 that preferably conforms with the tissue-contacting surface of the ablation electrode 24. Thus, because the tissue-contacting surface of the ablation electrode 24 is curved, the tissue-contacting surface 52 of each microelectrode 28 is likewise curved, with the radii of curvature for the respective surface being the same, thereby forming an electrode assembly with a substantially continuous surface (i.e., a surface with very little discontinuities or sharp edges). In this manner, RF energy will not be concentrated within localized regions of the ablation electrode 24 to create "hot spots" that would undesirably char tissue, which may otherwise occur at discontinuities. To ensure that the electrode assembly has a continuous external surface, the exterior surfaces of the ablation electrode 24 and microelectrodes 28 can be ground to a fine finish (e.g., #16 grit).

Referring to Fig. 4, the ablation electrode 24 comprises a plurality of holes 54 laterally extending through the wall 40 in communication with the bore 42, and the microelectrodes 28 are respectively disposed in the holes 54. The holes 54 may be formed by drilling through the wall 40 of the ablation electrode 24. Significantly, the microelectrodes 28 are electrically insulated from the ablation electrode 24, and thus, from each other, so that they can provide independent mapping channels. The microelectrodes 28 are also thermally insulated from the ablation electrode 24 to prevent saturation of the mapping channels that would otherwise cause interference from the heat generated during a radio frequency (RF) ablation procedure.

To this end, the ablation electrode 24 comprises a plurality of insulative bands 56 (best shown in Fig. 5) composed of the suitable electrically and thermally insulative material, such as a high temperature thermoset plastic with high dielectric properties, e.g., polyimide or plastics from the phenolic group, such as Bakelite® or Ultem® plastics. The insulative bands 56 are respectively mounted within the holes 54, and the microelectrodes 28 are mounted in the insulative bands 56. In this manner, the insulative bands 56 are interposed between the wall 40 of the ablation electrode 24 and the microelectrodes 28 to provide the desirable electrical and thermal insulation. The insulative bands 56 and microelectrodes 28 may be respectively mounted within the holes 54 using a suitable bonding material, such as, epoxy. An electrically and thermally insulative potting material 58 (such as a multicomponent (resin and hardener component) thermosetting or ultra-violet (UV)-curable resin, for example, silicone, urethane or epoxy) can also be introduced into the bore 42 of the ablation electrode 24 to ensure electrical insulation between the microelectrodes 28 and ablation electrode 24, to further secure the microelectrodes 28 to the ablation electrode 24, and to prevent cross-talk between the otherwise electrically insulated microelectrodes 28.

The electrophysiology catheter 14 further comprises a temperature sensor 60, such as a thermocouple or thermistor, which may be located on, under, abutting the longitudinal end edges of, or in the ablation electrode 24. In the illustrated embodiment, the temperature sensor 60 is mounted within a bore 42 formed at the distal tip of, and along the longitudinal axis of, the ablation electrode 24, as illustrated in Fig. 10, or, if a microelectrode 28 is incorporated into the distal tip of the ablation electrode 24, as illustrated in Figs. 7 and 8, within a bore 42 formed within, and along the longitudinal axis of, a microelectrode 28, as illustrated in Fig. 11. For temperature control purposes, signals from the temperature sensors are transmitted to the RF generator 18 via signal wires 62, so that RF energy to the ablation electrode 24 may be controlled based on sensed temperature. To this end, the signal wires 62 are passed in a conventional fashion through a lumen (not shown) extending through the associated catheter body 22, where they are electrically coupled either directly to the external connector 34 or indirectly to the external connector 34 via the PC board located in the handle assembly 30, which, in turn, Is electrically coupled to the RF generator 18 via the cable assembly 20.

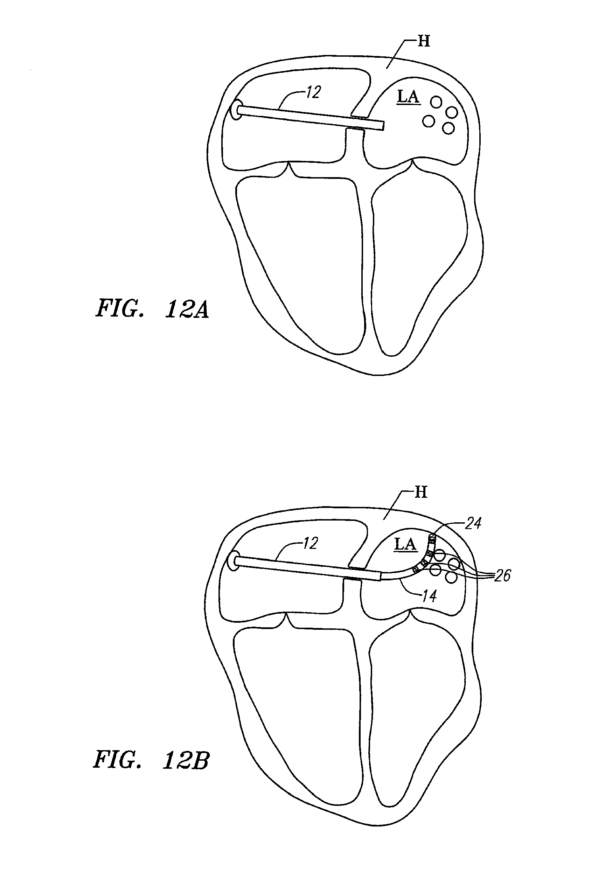

Having described the structure of the medical system 10, its operation in creating a lesion within the left atrium LA of the heart H to ablate or electrically isolate arrhythmia causing substrates will now be described with reference to Figs. 12A-12C. It should be noted that other regions within the heart H can also be treated using the medical system 10. It should also be noted that the views of the heart H and other interior regions of the body described herein are not intended to be anatomically accurate in every detail. The figures show anatomic details in diagrammatic form as necessary to show the features of the embodiment described herein.

First, the guide sheath 12 is introduced into the left atrium LA of the heart H, so that the distal end of the sheath 12 is adjacent a selected target site (Fig. 12A). Introduction of the guide sheath 12 within the left atrium LA can be accomplished using a conventional vascular introducer retrograde through the aortic and mitral valves, or can use a transeptal approach from the right atrium, as illustrated in Fig. 12A. A guide catheter or guide wire (not shown) may be used in association with the guide sheath 12 to aid in directing the guide sheath 12 through the appropriate artery toward the heart H.

Once the distal end of the guide sheath 12 is properly placed, the electrophysiology catheter 14 is introduced through the guide sheath 12 until its distal end is deployed from the guide sheath 12 (Fig. 12B). The steering mechanism 34 located on the handle assembly 30 (shown in Fig. 2) may be manipulated to place the ablation electrode 24 into firm contact with the endocardial tissue at a perpendicular angle to the wall of the heart H.

Once the ablation electrode 24 is firmly and stably in contact with the endocardial tissue, the mapping processor 16 (shown in Fig. 2) is operated in order to obtain and record ECG or MAP signals from the myocardial tissue via bipolar pairs of the microelectrodes 28 (shown in Fig. 2). These ECG or MAP signal measurements can be repeated at different locations within the left atrium LA to ascertain one or more target sites to be ablated. The user can analyze the ECGs or MAPs in a standard manner, or if electrical activity isochronal maps (whether or not combined with anatomical maps), can analyze these, to ascertain these target sites. Significantly, the use of the microelectrodes 28 substantially increases the resolution and enhances the fidelity of the ECG or MAP measurements. Alternatively, the mapping processor 16 can be operated to obtain and record ECG or MAP signals from the myocardial tissue via bipolar pairs of the ablation electrode 24 and mapping ring electrodes 26 if far field electrical potentials are desired; that is generalized mapping, in addition to highly localized mapping is desired.

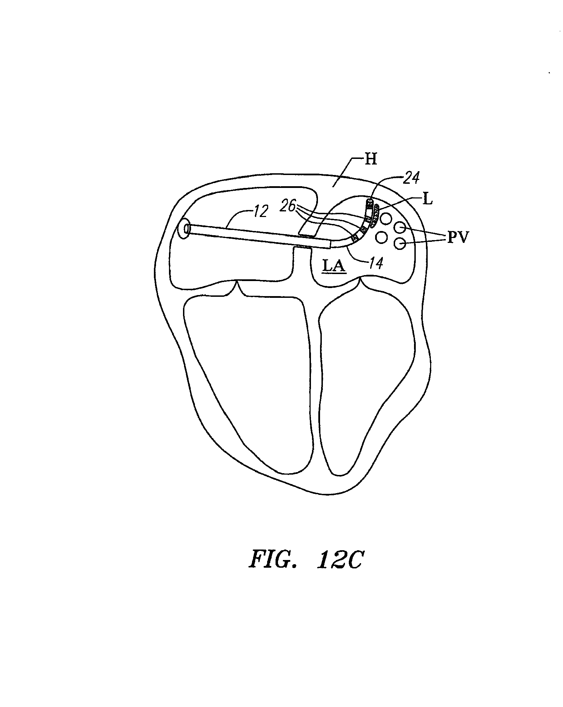

Once a target site has been identified via analysis of the ECG or MAP signals or isochronal electrical activity maps, the ablation electrode 24 is placed into firm contact with the target site, and the RF generator 18 (shown in Fig. 1) is then operated in order to convey RF energy to the ablation electrode 24 (either in the monopolar or bipolar mode), thereby creating a lesion L (Fig. 12C). Firm contact between the ablation electrode 24 and the endocardial tissue of the heart H can be confirmed by analyzing the ECG or MAP signals measured by the microelectrodes 28, with the amplitude of the ECG or MAP signals increasing as contact between the ablation electrode 24 and the endocardial tissue increases.

In the case where ablation is performed in or around the ostia PV of blood vessels, such as pulmonary veins or the superior vena cava, the contact with the endocardial tissue, as opposed to venous tissue, can be confirmed via analysis of the highly localized ECG or MAP signals measured by the microelectrodes 28. Ablation of the target site can be confirmed, again, by analyzing the highly localized ECG or MAP signals measured by the microelectrodes 28 during and after the ablation procedure, with the amplitude of the ECG or MAP signals gradually decreasing to zero as the tissue is successfully ablating. Significantly, since the microelectrodes 28 are incorporated into the ablation electrode 24, target site identification, electrode-tissue contact and characterization, tissue ablation, and lesion confirmation can all be performed without moving the ablation electrode 24.

To test the ability of the electrophysiology catheter 14 to record highly localized ECGs, a prototype was built to determine if the localized electrode-tissue contact is assessable with the localized ECG recordings, determine if the localized ECG recordings can be used as a lesion assessment tool, determine if the localized ECG recordings are stable during RF ablation energy delivery, and assess if the microelectrodes 28 undesirably create tissue char during RF ablation energy delivery. The ablation electrode 24 of the prototype 8mm long, and the four 0.070" diameter microelectrodes 28 were embedded around the ablation electrode 24 in a manner similar to that illustrated in Fig. 3.

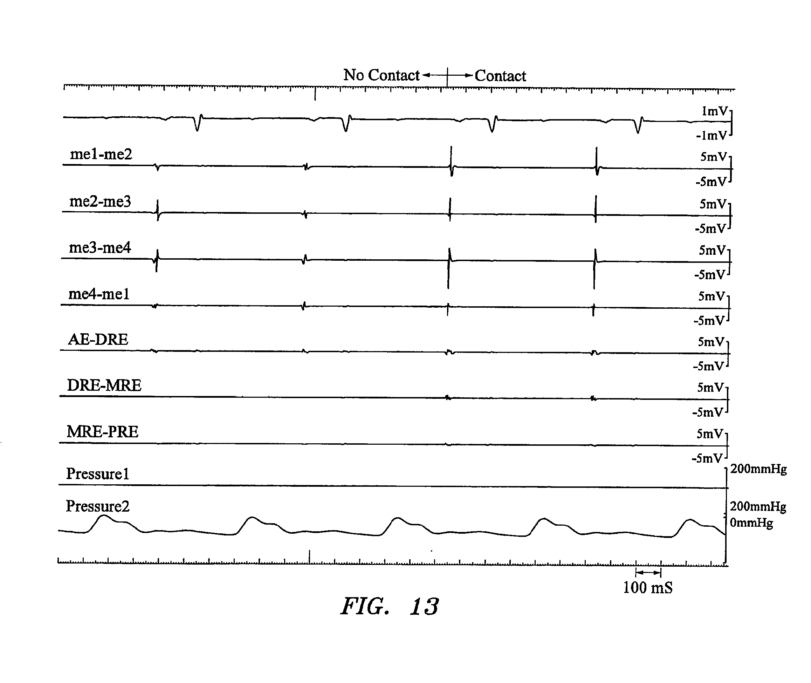

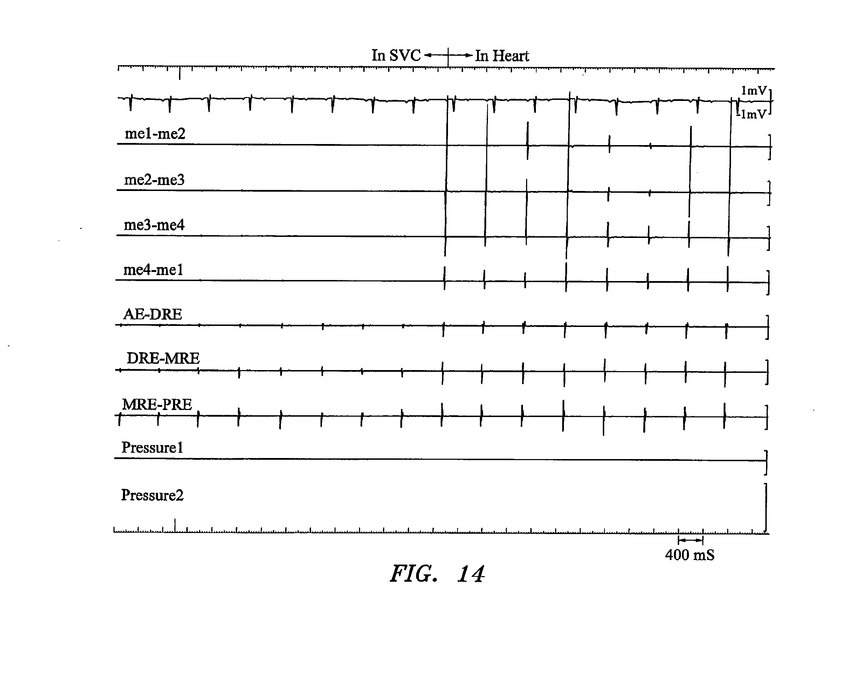

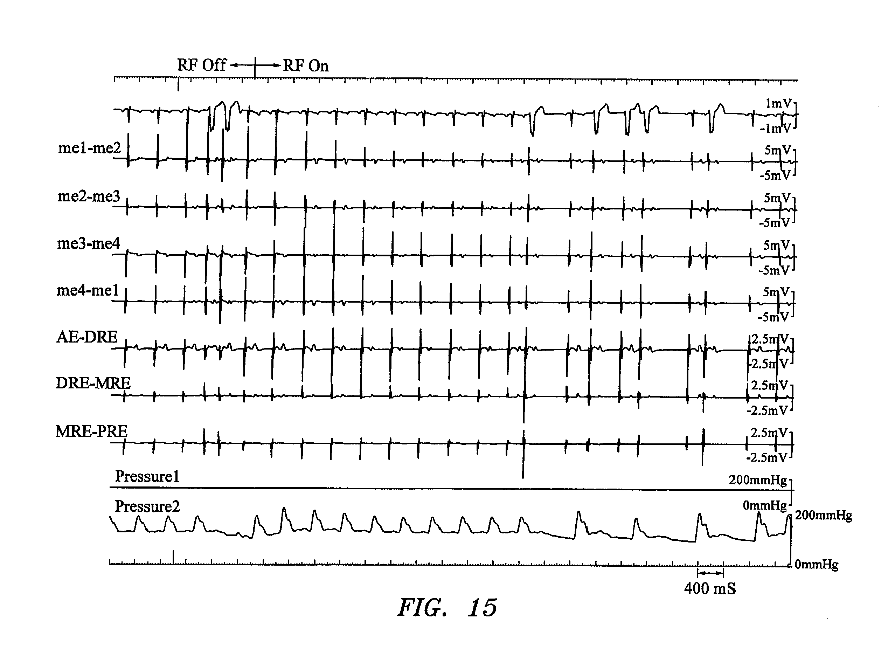

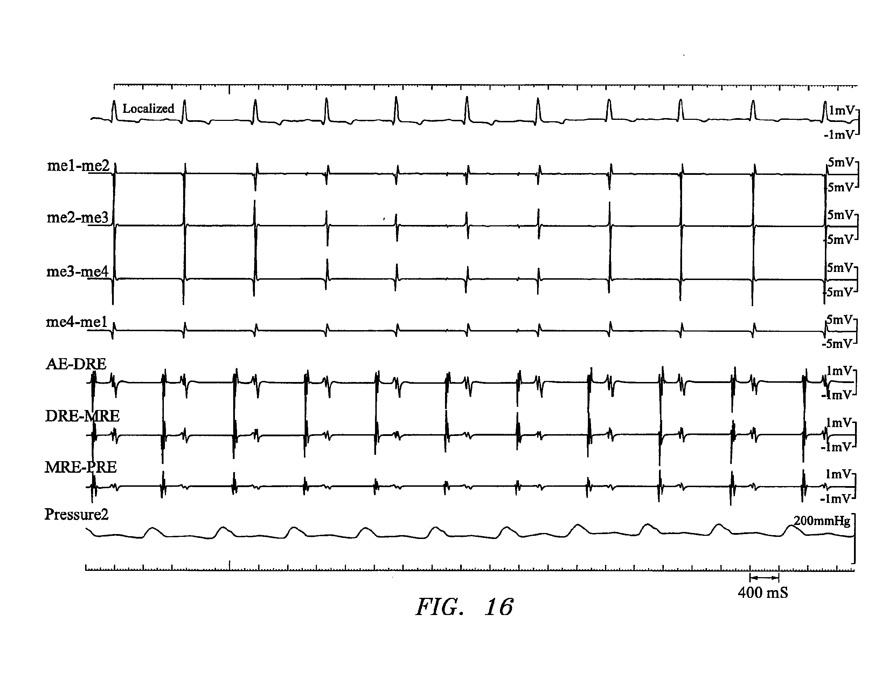

Tests of the prototype of the electrophysiology catheter 14 comparing the ECG measurements taken by the mapping microelectrodes 28 to ECG measurements taken by the mapping ring electrodes 26 were conducted in the right atrium of a dog. While recording ECGs with the microelectrodes 28 and ring electrodes 26, the distal end of the electrophysiology catheter 14 was (1) placed gradually into firm contact with the endocardial tissue via manipulation of the steering mechanism 34 (corresponding ECG tracings shown in Fig. 13); (2) placed into the superior vena cava and then slowly pulled into the right atrium (corresponding ECG tracings shown In Fig. 14); (3) operated to conduct an RF ablation in the right atrium (corresponding ECG tracings shown in Fig. 15); and (4) placed into contact with the right ventricle near the atrial-ventricular (AV) node (corresponding ECG tracings shown in Fig. 16). In each case, four bipolar ECG recordings were made by the four microelectrodes 28 (me1-me2, me2-me3, me3-me4, me4-me1), and three bipolar ECG recordings were made by the ablation electrode 24 and three ring electrodes 26 (ablation electrode-distal ring electrode (AE-DRE), distal ring electrode-medial ring electrode (DRE-MRE), and medial ring electrode-proximal ring electrode (MRE-PRE)).

As shown in Figs. 13-16, the microelectrodes 28 clearly separate the localized electrical activity at the ablation electrode 24 from the far field electrical activity that is normally associated with the ring electrode 26 measurements. That is, the higher resolution microelectrodes 28 generate very distinctly sharp, high amplitude, ECG tracings, compared to the typically slurred ECG tracings generated by the lower resolution ablation electrode 24 and ring electrodes 26.

As shown in Fig. 13, the amplitudes of the complexes of the ECG tracings recorded by the microelectrodes 28 increases as the contact between the ablation electrode and the tissue increases. In particular, the amplitudes of the ECG complexes recorded by the microelectrodes 28 become distinctly exaggerated when firm contact between the ablation electrode 24 and the tissue is achieved, in contrast to the ECG tracings recorded by the ablation/ring electrodes 24, 26, which have complexes of very low amplitudes during such firm contact that are virtually indistinguishable from the complexes when no contact between the ablation electrode and tissue occurs. As a result, the incorporation of microelectrodes within an ablation electrode proves to be a very useful tool for assessing electrode-tissue contact.

As shown in Fig. 14, the amplitudes of the complexes of the ECG tracings recorded by the microelectrodes 28 are essentially zero when the ablation electrode 24 is located within the superior vena cava, and then distinctly increase when the ablation electrode 24 is outside of the superior vena cava (SVC) in contact with the endocardial tissue. In contrast, the amplitudes of the complexes of the ECG tracings recorded by the ablation/ring electrodes 24, 26 are non-zero even when the ablation electrode 24 is located within the superior vena cava and do not substantially increase when the ablation electrode 24 is located outside of the superior vena cava in contact with the endocardial tissue. As discussed above, distinguishing between the endocardial tissue and venous tissue important when ablating in or around the ostia of pulmonary veins. Thus, the incorporation of microelectrodes within an ablation electrode proves to be a very useful tool for ensuring that an ablation procedure is not performed within a pulmonary vein.

As shown in Fig. 15, the amplitudes of the complexes of the ECG tracings recorded by the microelectrodes 28 significantly decrease about 5-10 seconds after initiation of RF energy delivery during an ablation procedure. Significantly, due to the proximity of the microelectrodes 28 to the ablation electrode 24, the changes to the complexes of the ECG tracings are very discernible during the ablation procedure. This is significant in that the distinct reduction of the amplitudes of the ECG tracings during ablation is a reliable indicator that the ablation electrode 24 is in firm contact with the tissue and that a lesion is forming. In contrast, the amplitudes of the complexes of the ECG tracings recorded by the ablation/ ring electrodes 24, 26 do not significantly change during the ablation procedure. Thus, the incorporation of microelectrodes within an ablation electrode proves to be a very useful tool for ensuring that the ablation procedure is efficiently creating a lesion within the myocardial tissue.

As shown in Fig. 16, the morphologies of the complexes of the ECG tracings are significantly different when recorded by the microelectrodes 28 and opposed to the ablation electrode/ring electrodes 24, 26, when the ablation electrode 24 is located in the ventricle adjacent the atrial-ventricular node. In particular, the ECG complexes recorded by the microelectrodes 28 reflect ventricular electrical activity, indicating that the ablation electrode 24 is located in the ventricle, whereas the ECG complexes recorded by the ablation /ring electrodes 24, 26 reflect both atrial and ventricular electrical activity, indicating that the ablation electrode 24 is located at the atrial-ventricular node, when in fact, it is not. Thus, the incorporation of microelectrodes within an ablation electrode proves to be a very useful tool for determining whether the ablation electrode is located in a region of the heart that can be distinguished from other regions of the heart based on the nature of electrical activity expected to be at the region.

## Claims

1. A medical system (10) comprising:
a medical probe comprising:
an elongated member having a distal end;
a metallic electrode (24) mounted to the distal end of the elongated member;
a plurality of microelectrodes (28) embedded within, and electrically insulated from, the metallic electrode;
a first wire (44) extending through the elongated member and connected to the metallic electrode; and
a plurality of second wires (48) extending through the elongated member and each connected to one of the plurality of microelectrodes;
a radio frequency,RF, ablation source (18) coupled to the first wire; and
a mapping processor (16) coupled to the plurality of second wires, wherein the mapping processor is configured to obtain and record electrocardiogram,ECG, or monophasic action potential,MAP, signals from myocardial tissue via bipolar pairs of the microelectrodes.

2. The medical system of claim 1, wherein the plurality of microelectrodes are configured as a plurality of the bipolar pairs of microelectrodes and the mapping processor is configured to preferably simultaneously obtain and record an ECG from each of the plurality of the bipolar pairs of microelectrodes.

3. The medical system of any of claims 1 or 2, wherein the system further comprises a display, wherein the mapping processor outputs the ECGs to the display.

4. The medical system of any of the previous claims, wherein the medical probe further comprises a plurality of ring electrodes (26). [based on p. 23, lines 1- 5 and p. 24, line 12 - p. 25, line 3]

5. The medical system of claim 4, wherein the plurality of ring electrodes is electrically coupled to the mapping processor via a further plurality of signal wires (46).

6. The medical system of any of claims 4 or 5, wherein the arrangement of the microelectrodes is configured such that a resolution of ECG measurements of the microelectrodes is higher than a resolution of ECG measurements of the ring electrodes.

7. The medical system of any of claims 4-6, wherein the microelectrodes are configured to generate sharp, high amplitude, ECG tracings, compared to the ECG tracings generated by the metallic electrode and the ring electrodes.

8. The medical system of any of the previous claims, wherein a diameter of each of the microelectrodes is equal to or less than half of a length of the metallic electrode.

9. The medical system of any of claims 1-7, wherein a diameter of each of the microelectrodes is equal to or less than a quarter of a length of the metallic electrode.

10. The medical system of any of the previous claims, wherein the metallic electrode has a cylindrical wall, and a bore surrounded by the cylindrical wall, and a plurality of holes extending through the cylindrical wall in communication with the bore, and wherein a plurality of electrically insulative bands respectively disposed in the holes, wherein the micro-electrodes are respectively disposed within the electrically insulative bands.

11. The medical system of any of the previous claims, wherein the medical probe further comprises a steering mechanism (34) located on a handle assembly (30), and the steering mechanism (34) is configured to place the metallic electrode into firm contact with cardiac tissue.

12. Use of a medical system according to any of the previous claims for assessing electrode-tissue contact.
